# EUROPEAN PATENT APPLICATION

(11) **EP 3 153 520 A1**
(43) Date of publication of application: **12.04.2017**
(21) Application number: 14893737.8
(22) Date of filing: 04.06.2014
(51) Int. Cl.: C07K 14/415, C07K 16/16, C12P 21/02, G01N 33/68

(54) **PEPTIDE TAGS FOR MARKING PROTEINS BY FUSION, AND ANTIBODIES FOR THE DETECTION THEREOF**

(71) Applicant: Abbcn, S.L., 08193 Bellaterra (ES); Immunostep, S.L., 37008 Salamanca (ES); Fundació Institut de Recerca Biomèdica (IRB Barcelona), 08028 Barcelona (ES); Instituto De Biología Molecular De Barcelona Consejo Superior De Investigaciones Cientificas, 08028 Barcelona (ES)
(72) Inventor: ALDEA MALO, Martí, E-08380 Malgrat De Mar (ES); OROZCO LÓPEZ, Modesto, E-08769 Castellví De Rosanes (ES); COSTA LEJA, Cristina, E-25199 Lleida (ES)
(74) Representative: ZBM Patents - Zea, Barlocci & Markvardsen
(86) International application number: PCT/ES2014/070460
(87) International publication number: WO 2015/185767

(57) **Abstract**

Peptide tags (tags) are provided for their fusion to proteins at the N-terminal or C-terminal ends, which are defined by a series of structural and functional properties that define their safety with regard to the modification of the structure or biological function of the protein or peptide to which they are fused, and wherein the tag-protein is selected from a group consisting of a fragment of Phl P 2 *Phleum pratense,* a fragment of Hev b 6.02 *Hevea brasiliensis,* and a fragment of Amb t 5 *Ambrosia trifida*, or a combination thereof. Methods are also provided for the production and detection of these recombinant fusion proteins as well as specific antibodies that bind to these tags.

LISTADO DE SECUENCIAS

<110> AbBCN S.L.
      Immunostep S.L.
      FUNDACIO INSTITUT DE RECERCA BIOMEDICA
      Instituto de Biologia Molecular de Barcelona - Consejo Super
      ior de Investigaciones Cientificas

<120> Etiquetas de marcaje - P2995PC00

<160> 5

<170> BiSSAP 1.0

<210> 1
<211> 96
<212> PRT
<213> Phleum pratense

<220>
<221> SOURCE
<222> 1..96
<223> /mol_type="protein"
      /note="Phl P 2 antigen as a tag"
      /organism="Phleum pratense"

<400> 1
Val Pro Lys Val Thr Phe Thr Val Glu Lys Gly Ser Asn Glu Lys His
1               5                   10                  15
Leu Ala Val Leu Val Lys Tyr Glu Gly Asp Thr Met Ala Glu Val Glu
            20                  25                  30
Leu Arg Glu His Gly Ser Asp Glu Trp Val Ala Met Thr Lys Gly Glu
        35                  40                  45
Gly Gly Val Trp Thr Phe Asp Ser Glu Glu Pro Leu Gln Gly Pro Phe
    50                  55                  60
Asn Phe Arg Phe Leu Thr Glu Lys Gly Met Lys Asn Val Phe Asp Asp
65                  70                  75                  80
Val Val Pro Glu Lys Tyr Thr Ile Gly Ala Thr Tyr Ala Pro Glu Glu
                85                  90                  95

<210> 2
<211> 43
<212> PRT
<213> Hevea brasiliensis

<220>
<221> SOURCE
<222> 1..43
<223> /mol_type="protein"
      /note="Hev b 6.02 as antigen tag"
      /organism="Hevea brasiliensis"

<400> 2
Glu Gln Cys Gly Arg Gln Ala Gly Gly Lys Leu Cys Pro Asp Asn Leu
1               5                   10                  15
Cys Cys Ser Gln Trp Gly Trp Cys Gly Ser Thr Asp Glu Tyr Cys Ser
            20                  25                  30
Pro Asp His Asn Cys Gln Ser Asn Cys Lys Asp
        35                  40

<210> 3
<211> 40
<212> PRT

<213> Ambrosia trifida

<220>
<221> SOURCE
<222> 1..40
<223> /mol_type="protein"
      /note="Amb t 5 as antigen tag"
      /organism="Ambrosia trifida"

<400> 3
Asp Asp Gly Leu Cys Tyr Glu Gly Thr Asn Cys Gly Lys Val Gly Lys
1               5                   10                  15
Tyr Cys Cys Ser Pro Ile Gly Lys Tyr Cys Val Cys Tyr Asp Ser Lys
            20                  25                  30
Ala Ile Cys Asn Lys Asn Cys Thr
        35                  40

<210> 4
<211> 16
<212> PRT
<213> Xenopus laevis

<220>
<221> SOURCE
<222> 1..16
<223> /mol_type="protein"
      /note="Nucleoplasmin"
      /organism="Xenopus laevis"

<400> 4
Lys Arg Pro Ala Ala Thr Lys Lys Ala Gly Gln Ala Lys Lys Lys Lys
1               5                   10                  15

<210> 5
<211> 4
<212> PRT
<213> Homo sapiens

<220>
<221> SOURCE
<222> 1..4
<223> /mol_type="protein"
      /organism="Homo sapiens"

<400> 5
Lys Asp Glu Leu
1

## Description

### TECHNICAL FIELD

The present invention relates to peptide tags (tags) and their fusion to recombinant proteins for its analysis, detection, separation and purification. It also relates to monoclonal antibodies that specifically bind to these peptide labeling tags. The invention has potential applications in various fields such as protein engineering, cell biology, and proteomics.

### STATE OF THE ART

Genetic engineering currently allows the manipulation and expression of virtually any DNA sequence (and the production of the protein it encodes) in an increasing number of host cells. Recombinant DNA technology allows two or more proteins to be fused so that the two are translated into a single polypeptide sequence. This is the fundamental idea on which protein tagging is based. A protein of interest may be labeled (fused) with a second antigenic tag protein for which there are specific antibodies (or another type of high affinity ligand). The protein of interest may thus be easily detected, separated or purified through the interaction of the fused antigen protein and its antibody or ligand.

Protein tagging is used in the separation and purification of proteins of therapeutic interest, and also in basic research in the field of cell and molecular biology. At present certain number of tags are available for labeling, including small peptides such as FLAG, c-Myc and HA, or proteins such as GFP or GST (Terpe K. "Overview of tag protein fusions: from molecular and biochemical fundamentals to commercial systems "Appl. Microbiol Biotechnol. 2003, vol. 60., pp. 523-533).

Peptide tags must meet strict standard requirements, especially if the goal is to use them in cellular studies *in situ.* Ideally, they should not alter the structure or interfere with the biological activity of the protein of interest, and must be stable in a variety of cellular contexts. They must also be accessible to their corresponding antibodies to promote easy detection, should not have a tendency to form complexes among themselves or other proteins, and must not alter or interfere with the localization, distribution and rate of degradation of the protein of interest.

Although some tags are more versatile than others, in general they do not all have the same reliability in all applications. Their efficiency usually fluctuates between acceptable to high range in the separation and purification of proteins, but their application in functional *in vivo* studies often continues to pose difficult problems because different labeling strategies always involve the risk of altering the function of the native protein due to unpredictable interactions with the peptide tag. Large tags are more likely to cause steric interference. Moreover, due to their intrinsic structural disorder, small tags can adopt many forms, which greatly facilitates their interaction with the target protein with unpredictable negative consequences on its function. First, spurious interactions of the tag in the intermediate folding phases can alter the final conformation of the target protein and favour its accumulation in large clusters. Secondly, the fusion protein may be more sensitive to degradation. Finally, if unwanted interactions take place with essential domains of the target protein, the tag can modify the structure, function, interactions, or localization. It is somewhat surprising that almost 20% of the 400 proteins analyzed in a recent integrative study showed different localization patterns when comparing tagging with fluorescent proteins to conventional immunofluorescence methods (Stadler, C. et al. "Immunofluorescence and fluorescent-protein tagging show high correlation for protein localization in mammalian cells" Nat. Methods 2013, vol. 10, pp. 315-323).

The limitations mentioned for tag-antibody pairs often represent an obstacle to research. Experimental studies that focus on accurately determining the localization, transport, compartmentalization and interaction of different proteins in the cell are often hampered by the lack of functionally innocuous tags and high-affinity antibodies so as to allow their use in *in situ* immunofluorescence experiments. In addition, by recommending a single tag-antibody pair for certain experimental approaches, researchers cannot try different tags to check if their presence, absence or swapping affects the appearance or disappearance of functions of the protein of interest, or the gain or loss of certain biological functions. Thus, there is a growing need in this field of the art to discover new tags with greater functional innocuousness and applicability in different experimental approaches, and whose antibodies show both greater sensitivity and less cross-reactivity.

### SUMMARY OF THE INVENTION

The inventors have found that labeling proteins of interest with certain peptide tags that meet a number of structural and functional requirements enables the detection of said proteins of interest even under the most demanding experimental conditions without compromising their original biological function. The peptide tag of the invention is selected from a group consisting of a fragment of Phl P 2 *Phleum pratense* (PDB 1WHP), a fragment of Hev b 6.02 *Hevea brasiliensis* (PDB 1WKX), and a fragment of Amb t 5 *Ambrosia trifida* (PDB 3BBG), or a combination thereof.

Surprisingly, it has been found that any of these three antigenic tags is highly innocuous in terms of alteration of the intrinsic properties of the tagged proteins. These tags, when fused in the same polypeptide chain with a wide variety of proteins, manage not to give rise to alterations in their diffusion, stability, cellular localization, degradation velocity, biological activity and other parameters, unlike other widely used tags in the field of the art such as HA or FLAG tags, which themselves often alter the functions of the proteins they label.

Therefore, the peptide tags of the invention allow cellular studies to be performed on inter-cellular expression, localization and/or traffic, protein-protein interaction or degradation of a variety of proteins with a much lower probability of altering their native behaviour, i.e. their behaviour when they are not labeled. This point will allow a breakthrough in the research of numerous proteins since, for the first time, they can be studied at cellular level (and/or purified under native conditions) without their behaviour being modified by the presence of the tag.

Thus, a first aspect of the invention is a recombinant fusion protein comprising a protein or peptide that is fused at its N-terminus or C-terminus end to a tag protein, where the tag protein is defined according to the following properties:
1.1) it comprises a number of amino acid residues below 100;
1.2) it is immunogenic;
1.3) it has a globularity index above 0.5, defining the globularity index as the fraction of residues with solvent accessibility and calculated using the Visual Molecular Dynamics (VMD) program;
1.4) it is free from enzymatic activity;
1.5) it is free from binding sites for cofactors or effectors;
1.6) it is free of degradation sequences;
1.7) it is free of localization sequences;
1.8) it is of vegetable origin;
1.9) it is free of homo-polymerization or hetero-polymerization sequences;
1.10) when the N-terminal end is fused to the Green Fluorescent Protein and the fusion protein is expressed in HEK293T cells, the resulting recombinant fusion protein maintains the expression levels which are measured by immunodetection; it maintains the structural stability defined according to immunodetection levels for 12 hours of treatment with 100 µg/ml cycloheximide; it maintains intracellular diffusion *in vivo* as measured by fluorescence loss in photobleaching, fluorescence measured by confocal fluorescence microscopy; and it maintains the solubility of GFP in cell extracts by centrifugation of extracts measured at 15,000 g for 15 minutes, *in vitro* and *in vivo*;
1.11) when it is fused to the C-terminal end of protein Cdc28 or protein Ydj1 of *Saccharomyces cerevisiae* the recombinant fusion protein maintains the morphology defined by bright-field microscopy; and maintains cell viability measured by plating at 42 °C;
1.12) when it is fused to the Green Fluorescent Protein and is expressed in *Escherichia coli*, it maintains the expression levels, where such levels are measured by immunodetection; it maintains the solubility of GFP in cell extracts measured by centrifuging extracts at 15,000 g for 15 minutes; and it maintains efficiency in purification by affinity chromatography measured by immunodetection;
1.13) it maintains the expression levels, where such levels are measured by immunofluorescence and maintains cell localization determined by confocal fluorescence microscopy of proteins that are found in different cell compartments, where such proteins are selected from the group consisting of: cytoskeleton beta-actin, protein STX6 of the Golgi apparatus, HO1 of the endoplasmic reticulum, nuclear HDAC2 and GRB2 of the plasma membrane in NIH3T3 mouse cells, as well as FMRP of the neuronal synapse in hippocampal neurons; and
where the tag-protein is selected from the group consisting of a fragment of Phl P 2 of *Phleum pratense* having an amino acid sequence with at least 70% identity to SEQ ID NO: 1, a fragment of Hev b 6.02 of *Hevea brasiliensis* having an amino acid sequence with at least 70 % identity to SEQ ID NO: 2 and a fragment of Amb t 5 of *Ambrosia trifida* having an amino acid sequence with at least 70 % identity to SEQ ID NO: 3, or a combination thereof.

A second aspect of the invention is a nucleotide sequence that encodes the recombinant fusion protein according to the first aspect of the invention.

A third aspect of the invention is a recombination expression cassette which comprises the nucleotide sequence according to the second aspect of the invention, functionally linked to an expression control sequence.

A fourth aspect of the invention is an expression vector that comprises a nucleotide sequence according to the second aspect of the invention or an expression cassette according to the third aspect of the invention.

A fifth aspect of the invention is a host cell that is transfected or transduced with the nucleotide sequence according to the second aspect of the invention.

A sixth aspect of the invention is a method for obtaining a recombinant fusion protein according to the first aspect of the invention, comprising:
1) Fusing at least one nucleotide sequence that encodes for the protein or peptide with a nucleotide sequence that encodes for the labeling tag;
2) Cloning the resulting sequence from step 1 in a host cell;
3) Expressing the sequence cloned in step 2 in a host cell;

A seventh aspect of the invention is a method for purifying or isolating a recombinant fusion protein according to the first aspect of the invention, which comprises carrying out the steps according to the sixth aspect of the invention and additionally a step wherein the fusion protein is purified or isolated.

An eighth aspect of the invention is a method for detecting a recombinant fusion protein according to the first aspect of the invention comprising the steps performed in the sixth aspect of the invention and additionally a step wherein means are added for detecting the tag protein.

It has also been found that when the recombinant fusion proteins that are part of the invention are detected by use of antibodies specifically developed against them, these allow for *in situ* cellular studies even of the proteins that have lower expression level and therefore require extremely high sensitivity for their detection.

A ninth aspect of the invention is a monoclonal antibody that specifically binds to a fragment of Phl P 2 of *Phleum pratense* having an amino acid sequence with at least 70 % identity to SEQ ID NO: 1, and which is produced by the hybridoma deposited on 27.03.2014 at the institution "Leibniz-Institut DSMZ - Deutsche Sammlung von Mikroorganismen und Zellkulturen GmbH." with the deposit number DSM ACC3234. The hybridoma of the invention was deposited under the Budapest Treaty on 27.03.2014, at the institution "Leibniz-Institut DSMZ - Deutsche Sammlung von Mikroorganismen und Zellkulturen GmbH", in Inhoffenstrasse 7 B, D-38124 Braunschweig, by the depositors AbBCN S.L. (Campus UAB 08193 Bellaterra) and Immunostep S.L. (Campus Unamuno, 37007, Salamanca). The hybridoma was identified by the depositor with reference ET5-1, and received access number DSM ACC3234 and was declared viable.

A tenth aspect of the invention is a monoclonal antibody that specifically binds to a fragment of Hev b 6.02 of *Hevea brasiliensis* having an amino acid with at least sequence 70 % identity to SEQ ID NO: 2, and which is produced by the hybridoma deposited on 14.05.2014 at the institution "Leibniz-Institut DSMZ - Deutsche Sammlung von Mikroorganismen und Zellkulturen GmbH." with the deposit number DSM ACC3242. The hybridoma of the invention was deposited under the Budapest Treaty on 14.05.2014, at the institution "Leibniz-Institut DSMZ - Deutsche Sammlung von Mikroorganismen und Zellkulturen GmbH", in Inhoffenstrasse 7 B, D-38124 Braunschweig, by the depositors AbBCN S.L. (Campus UAB 08193 Bellaterra) and Immunostep S.L. (Campus Unamuno, 37007, Salamanca). The hybridoma was identified by the depositor with reference ET6-1, and received access number DSM ACC3242 and was declared viable.

An eleventh aspect of the invention is a monoclonal antibody that specifically binds to a fragment of Amb t 5 of *Ambrosia trifida* having an amino acid sequence with at least 70 % identity to SEQ ID NO: 3, and which is produced by the hybridoma deposited on 27.03.2014 at the institution "Leibniz-Institut DSMZ - Deutsche Sammlung von Mikroorganismen und Zellkulturen GmbH." with the deposit number DSM ACC3236. The hybridoma of the invention was deposited under the Budapest Treaty on 27.03.2014, at the institution "Leibniz-Institut DSMZ - Deutsche Sammlung von Mikroorganismen und Zellkulturen GmbH", in Inhoffenstrasse 7 B, D-38124 Braunschweig, by the depositors AbBCN S.L. (Campus UAB 08193 Bellaterra) and Immunostep S.L. (Campus Unamuno, 37007, Salamanca). The hybridoma was identified by the depositor with reference ET10-1, and received access number DSM ACC3236 and was declared viable.

The combination of protein tags of the invention together with the specifically developed monoclonal antibodies represent a powerful tool for protein immunodetection, separation, isolation and purification applications requiring high sensitivity in the formation of antigen-antibody complex.

### BRIEF DESCRIPTION OF THE DRAWINGS

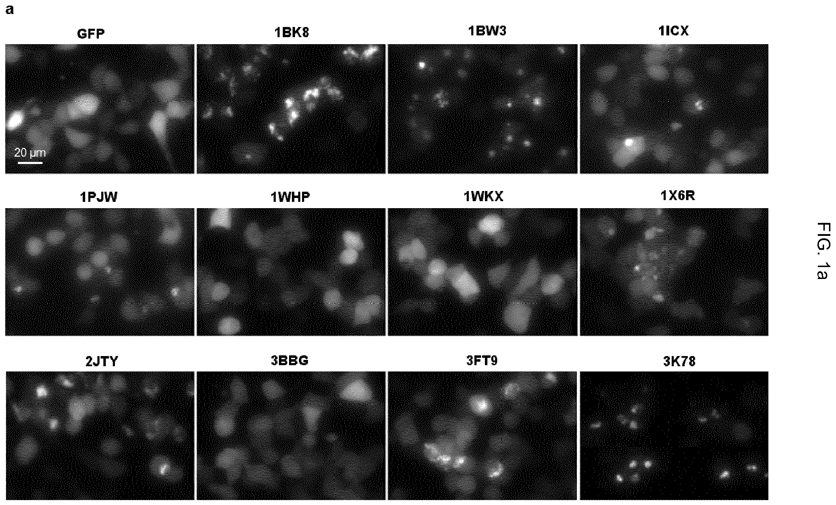

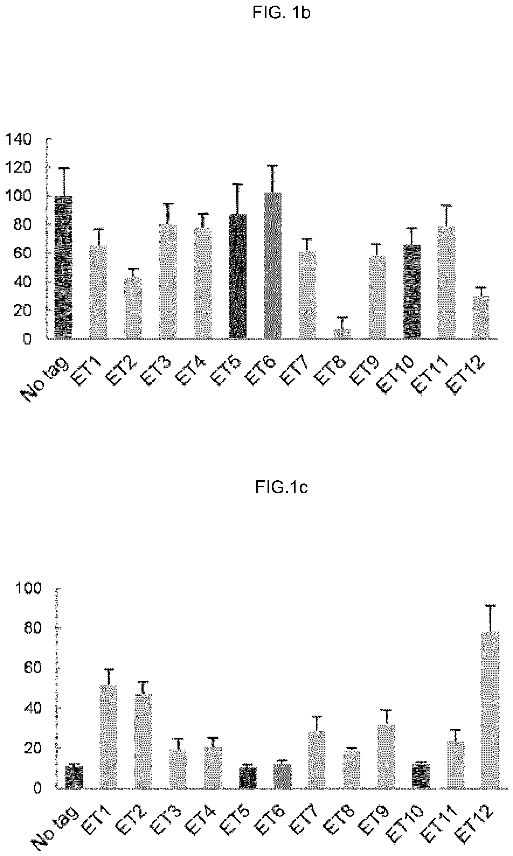

FIG. 1. Expression and aggregation tests of tags selected in the bioinformatic screening. a) Tags ET1 to ET12 were fused to GFP to analyze the effects on its expression and aggregation in HEK293T cells. The entry codes in the PDB database are indicated as a reference. The bar indicates 20 µm. b) The levels of expression the tag-GFP fusions are shown as mean values (30 cells) and confidence values for the mean (α= 0.05). The ordinate axis indicates expression units relative to the native GFP protein. c) As a measure of the degree of aggregation, the variation coefficient (%) of intracellular pixel values for the tag-GFP fusions tested was obtained, indicating the mean values (30 cells) and confidence intervals (α=0.05). The coordinate axis indicates the intracellular variability as a variation coefficient (%).

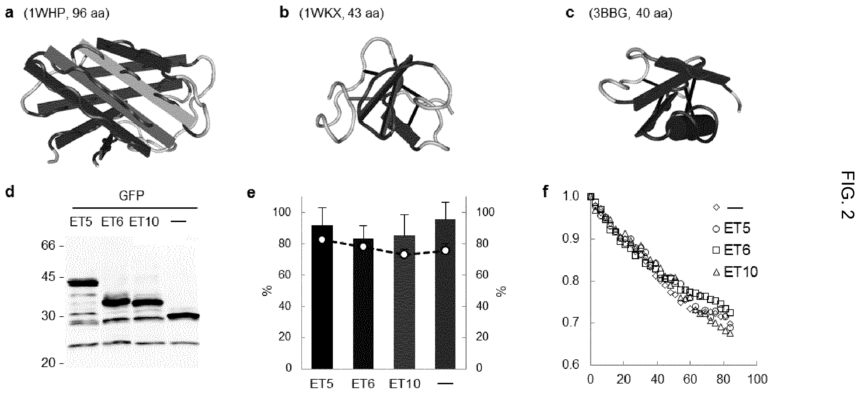

FIG.2. Solubility and stability tests for the tag proteins of the invention. Structural representation of ET5 (Phl P2, a), ET6 (Hev b 6.02, b) and ET10 (Hev t 5, c). d) The tag-GFP fusions were expressed in HEK293T cells and total extracts were analyzed by immunodetection with a GFP antibody. e) The levels of the tag-GFP fusions levels were analyzed 12 hours after the addition of cycloheximide by immunodetection and compared to untreated the control sample. Mean values (N = 3) and the confidence intervals for the mean (α= 0.05) are shown as bars (left axis). A soluble fraction of the HEK293T cells was obtained by centrifugation which expressed the tag-GFP fusion shown, and the levels obtained by immunoblotting were compared to those of the total cell extract. The mean values (N=3) and the mean confidence interval (α= 0.05) are indicated as open circles (right axis). f) The HEK293T cells that expressed the tag-GFP fusions shown were analyzed by confocal microscopy over time under photobleaching conditions (FLIP). The relative cellular fluorescence is shown at different times (in seconds) during the photobleaching.

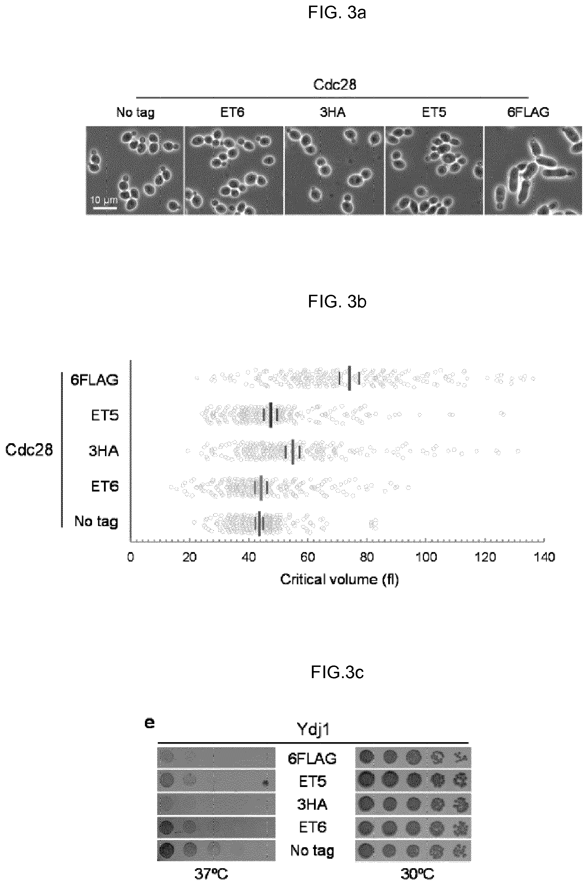

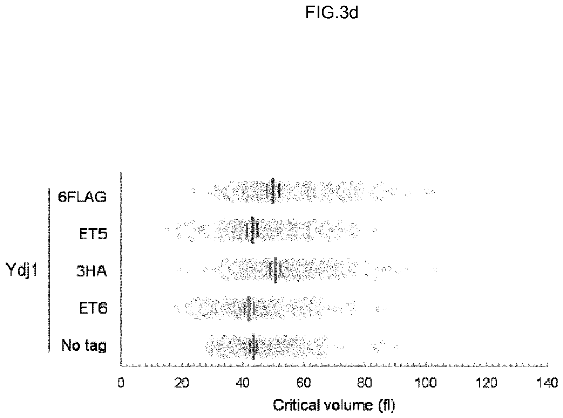

FIG.3. Functional innocuousness testing of the labeling tags of the invention and those of the state of the art Cdc28 and Ydj1, two essential proteins of budding yeast *S*. *cerevisiae.* a) Bright field images of yeast cells expressing endogenous levels of Cdc28 fused with ET5 (Phl P 2), ET6 (Hev b 6.02), 3HA and FLAG tags A control strain is shown (without tag) as a reference. The bar size is 10 µm. b) The graph shows the individual critical budding volumes of the cells occurring in section b (N>200). Mean values (thick vertical lines) and confidence intervals (α=0.05, thin vertical lines) are also shown. c) Serial dilutions of yeast cells expressing endogenous levels of Ydj1 fused to the ET5 (Phl P 2), ET6 (Hev b 6.02), 3HA and FLAG labeling tags were grown on plates and incubated at the indicated temperatures. A control strain (without labeling tag) is shown for reference. d) The graph shows the individual critical budding volumes of the cells occurring in section e (N>250). Mean values (thick vertical lines) and confidence intervals (α=0.05, thin vertical lines) are also shown.

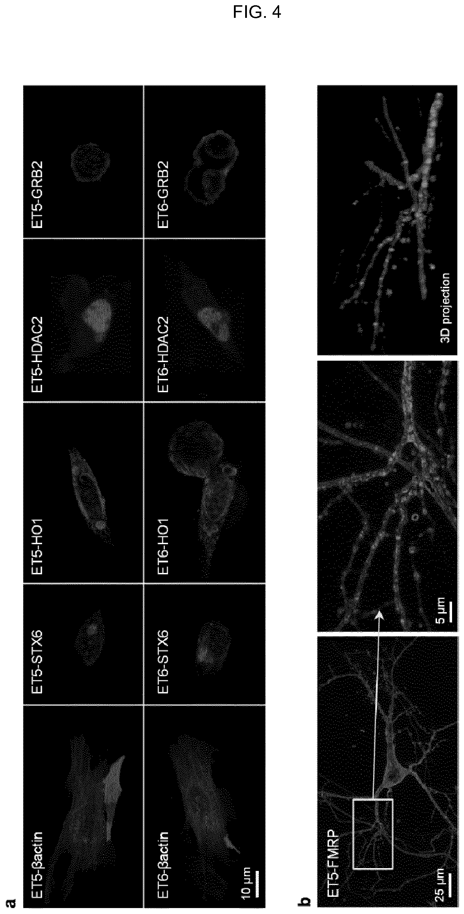

FIG. 4. Innocuousness of the labeling tags in the location of proteins that are found in different cell compartments: cytoskeleton (β-actin), Golgi (STX6), endoplasmic reticulum (HO1), nucleus (HDAC2), plasma membrane (GRB2), and neuronal synapses (FMRP). a) Tags ET5 (Phl P 2) and ET6 (Hev b 6.02) fused to the indicated proteins were expressed in NIH3T3 cells and analyzed by immunofluorescence (lighter signal in the figure) with corresponding αET5 and αET6 antibodies. The bar size is10 µm. b) The ET5-FMRP fusion was expressed in mouse hippocampus neurons and analyzed by immunofluorescence (lighter signal in the figure) with αET5 antibodies. An image of co-transfected GFP and treated to improve the somatic and neuritic thresholds is also shown for reference. Bar size is 25 bar µm. A 5x magnification of the indicated region is shown in the central panel. Bar size is 5 µm. In the right panel shows a 3D projection of this region.

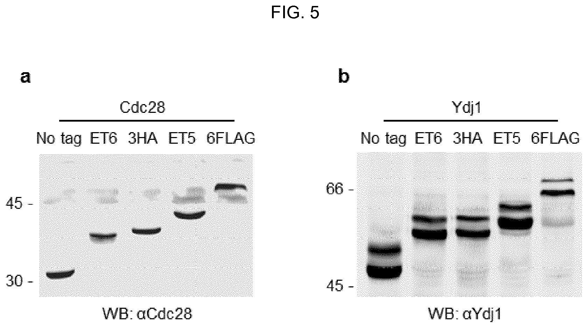

FIG. 5. Stability and integrity of the tags of the invention and other peptide tags commonly used when they are fused to essential proteins of budding yeast *S*. *cerevisiae.* Total extracts of yeast cells that express endogenous levels of Cdc28 (a) or of Ydj1 (b) fused to ET5 (Phl P 2), ET6 (Hev b 6.02), 3HA or 6FLAG were analyzed by immunodetection with αCdc28 or αYdj1, antibodies respectively. A control strain is also shown for reference.

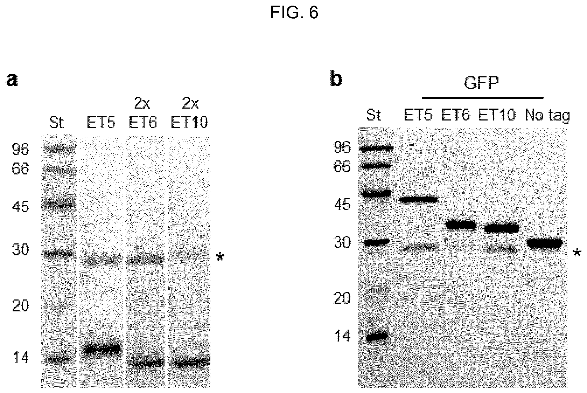

FIG. 6. Analysis of the integrity of the tags of the invention after their purification by affinity from cells of *E. coli.* a) The tags fused to 6His were purified by affinity chromatography for divalent metals, and were analyzed using SDS-PAGE. Due to their small size, tags ET6 and ET10 were expressed and purified as dimers. A common contaminant protein is labeled with an asterisk. b) The corresponding fusions to GFP-6His were expressed, purified and analyzed as in section a. The fusion of untagged GFP-6His is shown for reference.

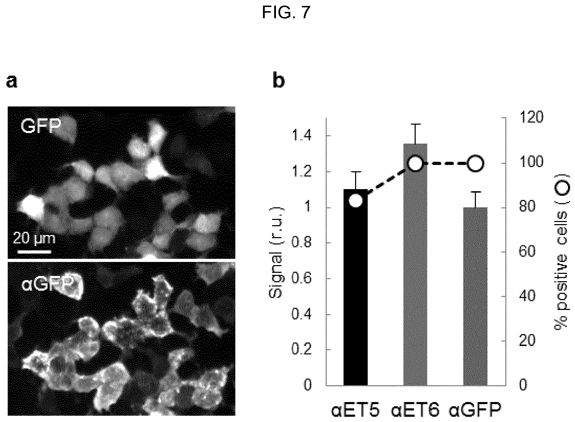

FIG. 7. Sensitivity analysis of antibodies that bind to the tags of the invention ET5 (Phl P 2) and ET6 (Hev b 6.02). a) The tags ET5 and ET6 fused to GFP were expressed in HEK293T cells and analyzed by immunofluorescence (lighter signal in the image of the bottom panel) with the corresponding αET5 and αET6 antibodies. The fluorescence of the GFP is also shown (clearer signal in the image of the top panel). The bar size is 20 µm. b) Quantification of immunofluorescence signals produced by selected αET5 and αET6 antibodies relative to those obtained with αGFP polyclonal antibody.
Mean values (>30 cells) and the confidence intervals for the mean (α= 0.05) are shown as bars (left axis). The percentages of GFP-positive cells that were also positive by immunofluorescence are also shown as open circles (right axis).

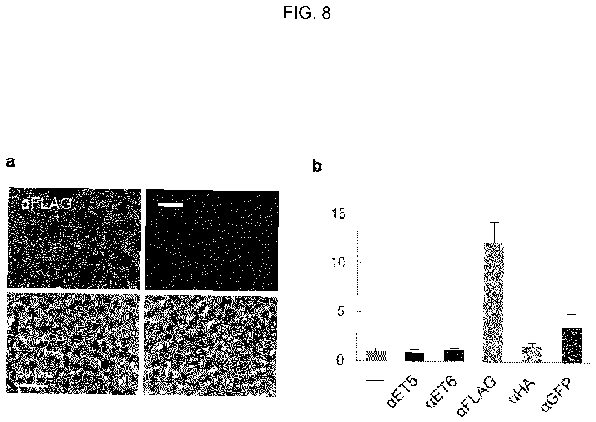

FIG. 8. Non-specific immunofluorescence signal of the monoclonal antibodies of the invention, against tags ET5 (Phl P 2) and ET6 (Hev b 6.02). a) An immunofluorescence analysis was performed of the HEK293T cells not transfected with the αFLAG antibody at a concentration 50 times above the usual to evaluate its non-specific binding to the different cellular components (top-left panel). Cells incubated only with the secondary antibody were used as a control (top-right panel). The bright-field images of the corresponding fields are also shown (lower panels). Bar size is 50 µm. b) Quantification of the non-specific immunofluorescence signal produced by the αET5, αET6, αFLAG, αHAS, and αGFP antibodies had a concentration 50 times above usual. The values are relativized to the value obtained with the secondary antibody. The mean values (> 30 cells) and confidence intervals for the mean (α=0.05) are also shown

### DETAILED DESCRIPTION OF THE INVENTION

One aspect of this invention is a recombinant fusion protein comprising a protein or peptide that is fused at its N-terminus or C-terminus end to a tag protein, wherein the tag protein is defined according to the following properties:
1.1) it comprises a number of amino acid residues below 100;
1.2) it is immunogenic; 1.3) it has a globularity index above 0.5, defining the globularity index as the fraction of residues with solvent accessibility and calculated using the VMD program;
1.4) it is free from enzymatic activity; 1.5) it is free from binding sites for cofactors or effectors; 1.6) it is free of degradation sequences;
1.7) it is free of localization sequences; 1.8) it is of vegetable origin;
1.9) it is free of homo-polymerization or hetero-polymerization sequences;
and where the tag-protein is selected from the group consisting of a fragment of Phl P 2 of *Phleum pratense* having an amino acid sequence with at least 70 % identity to SEQ ID NO: 1, a fragment of Hev b 6.02 of *Hevea brasiliensis* having an amino acid sequence with at least 70 % identity to SEQ ID NO: 2 and a fragment of Amb t 5 of *Ambrosia trifida* having an amino acid sequence with at least 70 % identity to SEQ ID NO: 3, or a combination thereof.

As described above,
a first aspect of the invention is a recombinant fusion protein comprising a protein or peptide that is fused at its N-terminus or C-terminus end to a tag protein where the tag protein is defined according to the following properties:
1.1) it comprises a number of amino acid residues below 100; 1.2) it is immunogenic; 1.3) it has a globularity index above 0.5, defining the globularity index as the fraction of residues with solvent accessibility and calculated using the VMD program; 1.4) it is free from enzymatic activity; 1.5) it is free from binding sites for cofactors or effectors;
1.6) it is free of degradation sequences; 1.7) it is free of localization sequences; 1.8) it is of vegetable origin; 1.9) it is free of homo-polymerization or hetero-polymerization sequences; 1.10) when the N-terminal end is fused to the Green Fluorescent Protein and the fusion protein is expressed in HEK293T cells, the resulting recombinant fusion protein maintains the expression levels which are measured by immunodetection; it maintains the structural stability defined according to immunodetection levels for 12 hours of treatment with 100 µg/ml of cycloheximide; it maintains intracellular diffusion *in vivo* as measured by fluorescence loss in photobleaching, whereby the fluorescence is measured by confocal fluorescence microscopy; and it maintains the solubility of GFP in cell extracts by centrifugation of extracts measured at 15,000 g for 15 minutes, *in vitro* and *in vivo*; 1.11) when it is fused to the C-terminal end of protein Cdc28 or protein Ydj1 of *Saccharomyces cerevisiae* the recombinant fusion protein maintains the morphology defined by bright-field microscopy; and maintains cell viability measured by plating at 42 °C; 1.12) when it is fused to the Green Fluorescent Protein and is expressed in *Escherichia coli,* it maintains the expression levels, where such levels are measured by immunodetection; it maintains the solubility of GFP in cell extracts measured by centrifuging extracts at 15,000 g for 15 minutes; and it maintains efficiency in purification by affinity chromatography measured by immunodetection; 1.13) it maintains the expression levels, where such levels are measured by immunofluorescence and maintains cell localization determined by confocal fluorescence microscopy of proteins that are found in different cell compartments, where such proteins are selected from the group consisting of: cytoskeleton beta-actin, protein STX6 of the Golgi apparatus, HO1 of the endoplasmic reticulum, HDAC2 nuclear and GRB2 of the plasma membrane in NIH3T3 mouse cells, as well as FMRP of the neuronal synapse in hippocampus neurons; and
wherein the tag-protein is selected from the group consisting of a fragment of Phl P 2 of *Phleum pratense* having an amino acid sequence with at least 70 % identity to SEQ ID NO: 1, a fragment of Hev b 6.02 of *Hevea brasiliensis* having an amino acid sequence with at least 70 % identity to SEQ ID NO: 2 and a fragment of Amb t 5 of *Ambrosia trifida* having an amino acid sequence with at least 70 % identity to SEQ ID NO: 3, or a combination thereof.

In a specific embodiment of the first aspect of the invention, the tag-protein is a fragment of Phl P 2 of *Phleum pratense* having an amino acid sequence with at least 70 % identity to SEQ ID NO: 1

In a specific embodiment of the first aspect of the invention, the tag-protein is a fragment of Phl P 2 of *Phleum pratense* having an amino acid sequence with at least 80 % identity to SEQ ID NO: 1

In a specific embodiment of the first aspect of the invention, the tag-protein is a fragment of Phl P 2 of *Phleum pratense* having an amino acid sequence with at least 90 % identity to SEQ ID NO: 1 In another specific embodiment of this first aspect of the invention, the tag-protein is a fragment of Phl P 2 of *Phleum pratense* having an amino acid sequence consisting of SEQ ID NO: 1, or what amounts to the same, having 100 % identity with SEQ ID NO: 1.

In another specific embodiment of the first aspect of the invention, the tag-protein is a fragment of Hev b 6.02 of *Hevea brasiliensis* having an amino acid sequence with at least 70 % identity to SEQ ID NO:2.

In another specific embodiment of the first aspect of the invention, the tag-protein is a fragment of Hev b 6.02 of *Hevea brasiliensis* having an amino acid sequence with at least 80 % identity to SEQ ID NO:2.

In another specific embodiment of the first aspect of the invention, the tag-protein is a fragment of Hev b 6.02 of *Hevea brasiliensis* having an amino acid sequence with at least 90 % identity to SEQ ID NO: 2. In another specific embodiment of this first aspect, the tag-protein is a fragment of Hev b 6.02 of *Hevea brasiliensis* having an amino acid sequence iconsisting of SEQ ID NO:2. or what amounts to the same, having 100 % identity with SEQ ID NO: 2.

In another specific embodiment of the first aspect of the invention, the tag-protein is a fragment of Amb t 5 of *Ambrosia trifida* having an amino acid sequence with at least 70 % identity to SEQ ID NO: 3.

In another specific embodiment of the first aspect of the invention, the tag-protein is a fragment of Amb t 5 of *Ambrosia trifida* having an amino acid sequence with at least 80 % identity to SEQ ID NO: 3.

In another specific embodiment of the first aspect of the invention, the tag-protein is a fragment of Amb t 5 of *Ambrosia trifida* having an amino acid sequence with at least 90 % identity to SEQ ID NO: 3. In another specific embodiment of this first aspect, the tag-protein is a fragment of Amb t 5 of *Ambrosia trifida* having an amino acid sequence consisting of SEQ ID NO: 3, or what amounts to the same, having 100 % identity with SEQ ID NO: 3.

All these sequences of the tag-proteins fused to the protein or peptide of interest are coded, according to the second aspect of the invention, by a nucleotide sequence.

As described above, a third aspect of the invention is a recombination expression cassette which comprises the nucleotide sequence according to the second aspect of the invention, functionally linked to an expression control sequence.

In a specific embodiment of the third aspect of the invention, the expression cassette is such that the control of the expression sequence comprises a target sequence for transcriptional regulators, a ribosome binding sequence and a transcription termination sequence.

As described above, a seventh aspect of the invention is a method for purifying or isolating a recombinant fusion protein according to the first aspect of the invention, which comprises carrying out the steps according to the sixth aspect of the invention and additionally a step wherein the fusion protein is purified or isolated.

In a specific embodiment of the seventh aspect of the invention, the additional purification or isolation step of the fusion protein includes an antibody or fragment thereof that binds to the tag-protein.

As described above, an eighth aspect of the invention is a method for detecting a recombinant fusion protein according to the first aspect of the invention that comprises performing the steps according the sixth aspect of the invention and additionally a step wherein means are added for detecting the tag-protein.

In a specific embodiment of the eighth aspect of the invention, the means for detecting the tag-protein include an antibody or a fragment thereof.

In another specific embodiment of the eighth aspect of the invention, the means for detecting the tag-protein include a monoclonal antibody.

In a specific embodiment of the seventh and eighth aspects of the invention, the means are selected from the antibodies that are obtained by the hybridoma deposited in the institution "Leibniz-Institut DSMZ - Deutsche Sammlung von Mikroorganismen und Zellkulturen GmbH" with deposit number DSM ACC3236 (deposited on 27.03.2014), DSM ACC3234 (deposited on 27.03.2014) and DSM ACC3242 (deposited on 14.05.2014).

Throughout the description and claims the word "comprises" and its variants are not meant to exclude other technical characteristics, additives, components or steps. In addition, the word "comprises" includes the case "consists of". For experts in the field, other objects, advantages and features of the invention will be become apparent in part from the description and in part from the embodiments of the invention. The following examples are provided by way of illustration, and they are not intended to be limiting of the present invention. In addition, the present invention covers all the possible combinations of specific and preferred embodiments contained herein.

### Definitions

All terms used in this application are to be understood by their commonly used meaning in the field of the art. However, in order to improve the clarity, the following definitions are included. The abbreviations of the proteins cited in this application are also listed below, together with their entry in the Uniprot database.

The term "recombinant fusion protein" as used herein means a single chain polypeptide that is composed of: 1) a protein of interest (protein used for studying cell expression, distribution, compartmentalization, interaction or protein that will be isolated, purified or separated), and 2) a tag-protein.

The terms "tag-protein" or "labeling tag" (terms that are interchangeable in this application and also named as "tags") as used herein mean a protein for which there are antibodies or other high affinity ligands, that is expressed fused (in the same polypeptide chain) to a protein of interest in such a way that the protein of interest can be separated, purified or localised in cell studies thanks to the interaction of the tag-protein with its high-affinity ligand.

The term "immunogenic" as used herein, means a substance (in the case of the present invention, a protein) that is capable of inducing an immune response.

The term "globularity index" gives an idea of the compactness of a protein's structure, and is related to its stability and structural integrity. It is known that numerous proteins (or some of their domains) are intrinsically disordered, i.e., they do not acquire a fixed three-dimensional structure. The more disorderly a protein is, the lower its globularity, and it is defined as the fraction of residues with accessibility to the solvent. In a disordered protein, the average of the accessibility to the solvent of its residues increases. There are numerous programs that calculate the accessibility to the solvent of protein residues. In this invention, the program VMD has been used.

The term "degradation sequence" as used herein means an amino acid sequence that, when present in a polypeptide chain, directs the latter to a degradation or partial proteolysis pathway. For example, a degradation sequence commonly known and referred to as PEST comprises segments rich in proline, glutamic acid, serine and threonine.

The term "localization sequence" as used herein means an amino acid sequence that, when present in a polypeptide chain, directs the latter to a particular location within cellular compartments (such as the Golgi apparatus, the endoplasmic reticulum, the nucleus of the cell, etc.) For example, a typical nuclear localization sequence is the sequence KRPAATKKAGQAKKKK (SEQ ID NO: 4) of nucleoplasmin, which is composed of two groups of positively charged residues, and that is recognised by the importin-α; a typical sequence of the C-terminal end resulting in the retention of the protein in the endoplasmic reticulum is KDEL (SEQ ID NO: 5).

The term "homo-polymerization or hetero-polymerization sequence" as used herein means a sequence that, when present in a polypeptide chain, forms appropriate complexes with other different polypeptide chains that are either identical (homo) or different with regard to sequence or structure (hetero).

The term "effector" as used herein is a protein, a small organic molecule (such as a hormone, a metabolite, etc.) or an ion, which selectively binds to a protein to regulate its function. The effector usually has a specific bonding site in the protein that it regulates.

The term "free of' as used herein, means "devoid of".

The term "free of enzyme activity" used herein means that the protein does not catalyse any biochemical reactions.

The term "structural stability" used herein means that the protein does not lose the three-dimensional conformation responsible for its biological activity, it is not degraded, metabolised, or eliminated through different protein processing cell systems.

The term "transduction" as is used herein, means a process by which foreign DNA (heterologous) is introduced into a host cell through the use of a viral vector. The ultimate goal of this process is for the foreign DNA to become integrated into the genome of the host cell in such a way that the encoding RNA and/or protein is stably expressed in this cell. In the case of the present invention, transduction occurs with the encoding DNA for the recombinant fusion proteins.

The term "transfection" as is used herein, means a process by which foreign DNA (heterologous) is introduced into a host cell through any non-viral method, such as electroporation or by chemical treatment. In the case of the present invention, transfection occurs with the encoding DNA for the recombinant fusion proteins.

As used in this description, the expression 'operationally linked' means that the product or products of interest is/are expressed in the correct reading frame under the control of the expression control or regulator sequences.

In the present invention, the term "percentage of identity" means the percentage of amino acids or nucleotides that are the same between two or more independent sequences that are compared or aligned in such a way as to obtain the best value of coincidences between positions. The percent identity between peptide sequences and between nucleotide sequences are preferably calculated using the BLAST algorithm by "*Basic Local Alignment Search Tool*", accessible on the NCBI website (http://www.ncbi.nlm.nih.gov/BLAST) and described in Altschul S. "Basic local alignment search tool", J. Mol. Biol. 1990, vol. 215, pp. 403-410 Thus when in the present invention it is indicated that two sequences have at least 70 % identity, it means that once aligned, the two sequences have at least 7 out of 10 identical amino acids.

In the sense of the present invention, the term "antibody or fragment thereof", means any polyclonal or monoclonal antibody, or fragment derivative thereof which still retains the ability to bind its antigen. Examples of fragments are: a (Fab)2 fragment derived from digestion of an antibody with pepsin, a Fab fragment derived from digestion of an antibody with papain, a scFv fragment derived from the fusion of two variable domains of the heavy and light chains, nanobodies derived from camelid or shark antibodies etc.

Green Fluorescent Protein, or "GFP" is a protein of 238 residues isolated from the jellyfish *Aequorea victoria,* which has a strong green fluorescence when exposed to ultraviolet light. This protein is commonly used as a marker in molecular and cellular biology studies. Since it is easily detectable it is used to control the level of genetic expression, to control cell development, division in prokaryotes, etc. It is located in the Uniprot database under entry (code) P42212, version 118 of the entry of 14 May 2014 version 1 of the sequence of 1 November 1995.

Cdc28 of *Saccharomyces cerevisiae* (or "cell division control protein 28" also called cyclin dependent kinase 1), is an essential protein for yeast cell cycle control. It is located in the Uniprot database under entry (code) P00546, version 160 of the entry of 14 May 2014 and version 1 of the sequence of 21 July 1986.

Ydj1 of *Saccharomyces cerevisiae* (or "yeast DNAJ protein 1", also known as "mitochondrial protein import protein MA S5") is a protein of 409 amino acids involved in the import of proteins to yeast mitochondria. It is located in the Uniprot database under entry (code) P25491, version 146 of the entry of 14 May 2014 and version 1 of the sequence of 1 May 1992.

Beta-actin of *Mus musculus* is a protein of 375 residues which is involved in various processes of cell motility and that forms an integral part of the cytoskeleton. It is located in the Uniprot database under entry (code) P60710, version 110 of the entry of 14 May 2014 and version 1 of the sequence of 1 April 1988.

STX6 of *Mus musculus* (also called Sintaxin-6) is a protein of 255 residues involved in intracellular vesicular trafficking. It is located in the Uniprot database under entry (code) Q9JKK1, version 101 of the entry of 14 May 2014 and version 1 of the sequence of 1 October 2000.

HO1 of *Mus musculus* (Heme oxigenase 1, also called protein P32) is a protein of 289 residues that is located in the endoplasmic reticulum, and that is involved in breaking of the heme group ring. It is located in the Uniprot database under entry (code) P41901, version 119 of the entry of 14 May 2014 and version 1 of the sequence of 1 April 1990.

HDAC2 of *Mus musculus* (also called HD2, Histone deacetylase 2) is a protein of 488 residues located in the cell nucleus and that is involved in the deacetylation of lysine residues in the N-terminal end of histones (H2A, H2B, H3 and H4), and is therefore involved in the packaging of DNA and in the epigenetic regulation of the expression of numerous genes. It is located in the Uniprot database under entry (code) P70288, version 142 of the entry of 14 May 2014 and version 1 of the sequence of 01 February 1997.

GRB2 of *Mus musculus* (growth factor receptor-bound protein2, also called "Adapter protein GRB2") is a protein of 217 residues which can be located in the cytoplasm, in endosomes or in the Golgi apparatus, and plays a role in the connection between surface growth factors and the Ras signalling pathway. It is located in the Uniprot database under entry (code) Q60631, version 155 of the entry of 14 May 2014 and version 1 of the sequence of 01 November 1996.

FMRP of *Mus musculus* (Fragile X mental retardation protein) is a protein of 589 residues that are found in hippocampal neurons. It is located in the Uniprot database under entry (code) Q547R0, version 51 of the entry of 16 April 2014 and version 1 of the sequence of 24 May 2005.

The term "NIH3T3" refers to the cell line also called 3T3. It is standard fibroblast cell line widely used in cell research. This line was established in 1962 by two researchers at the University of New York, George Todaro and Howard Green.

"Phl P 2" is the allergen from the pollen Phl P 2 derived from *Phleum pratense,* a perennial herb that grows across most of Europe. It is located in the Uniprot database under entry (code) P43214, (last updated 16 October 2013, version 18 of the entry and version 1 of the sequence of 1 November 1995). Its three-dimensional structure has been determined by X-ray crystallography and is available in the Protein Data Bank (PDB) with code 1 WHP. The amino acid sequence of the fragment of the allergen Phl P 2 used in the present invention is (SEQ ID NO 1):
VPKV TFTVEKGSNE KHLAVLVKYE GDTMAEVELR EHGSDEWVAM TKGEGGVWTF DSEEPLQGPF NFRFLTEKGM KNVFDDVVPE KYTIGATYAP EE

"Hev b 6.02" is an allergen derived from *Hevea brasiliensis* (rubber tree), the most important species of trees producing latex in South America. It is located in the Uniprot database under entry (code) Q6JYQ7, (last updated 19 February 2014, version 51 of the entry and version 1 of the sequence of 5 July 2004). Its three-dimensional structure has been determined by X-ray crystallography and is available in the Protein Data Bank (PDB) with code 1 WKX. The amino acid sequence of the fragment of the allergen Hev b 6.02 used in the present invention is (SEQ ID NO 2):
EQCGRQAGGKLCPDNLCCSQWGWCGSTDEYCSPDHNCQSNCKD

"Amb t 5" is the Amb t 5 pollen allergen derived from *Ambrosia trifida* (a species of the Ambrosia genus, a herbaceous plant very common in America). It is located in the Uniprot database under entry (code) P10414, (last updated 19 February 2014, version 82 of the entry and version 2 of the sequence of 1 August 1992). Its three-dimensional structure has been determined by nuclear magnetic resonance and is available in the Protein Data Bank (PDB) with code 1 3BBG. The amino acid sequence of the fragment Amb t 5 used in the present invention is (SEQ ID NO 3):
DDGLCYEGTNCGKVGKYCCSPIGKYCVCYDSKAICNK NCT

### EXAMPLES

### Material and Methods

### Bioinformatic screening.

The initial aim of the bioinformatic screening was to obtain a list of protein domains suitable for preparing fusion proteins. Due to the difficulty of predicting antigenicity based on purely theoretical approaches, the screening began with around 2000 families of proteins reported in the VarDB database (Hayes, C. N. et al. "varDB: a pathogen-specific sequence database of protein families involved in antigenic variation" Bioinformatics 2008, vol. 24, pp. 2564-2565) and epitopes reported in the IEDB database (Vita, R. et al "The immune epitope database 2.0" Nucl. Acids Res. 2010, vol. 38, D854-D862). The structures of proteins containing the 63,424 epitopes listed in the IEDB were obtained using BLASTP against the PDB (standard parameters were used). Finally, the list was extended to include possible homologues using the corresponding PFAM domains (Punta, M. et.al. "The Pfam protein families database". Nucl. Acids Res. 2012, vol. 40, D290-D301). The final list included 3,500 antigenic proteins which covered 1,383 PFAM domains.

The initial list was screened to check the following properties:
- Antigenicity using BCIPRED (Saha, S. & Raghava, G. P. S. "BcePred: prediction of continuous B-cell epitopes in antigenic sequences using physico-chemical properties" in Nicosia, G., Cutello, V., Bentley, P.J. & Timis, J. (eds.) pp. 197-204, Springer 2004, Heidelberg). The method uses a combination of estimates based on sequences including: hydrophilicity, flexibility, accessibility, trend to form beta turns, antigenic propensity and polarity. The domains considered as antigenic according to at least two of the above criteria, were accepted.
- Accessibility to the solvent, the distance of the epitope to the N and C-terminal ends, the accessibility of the epitope to the solvent and the globularity index were calculated with VMD (Humphrey, et.al. "VMD - Visual molecular dynamics" J. Molec. Graphics 1996, vol. 14, pp. 33-38.)
- The functional characterization was obtained from the PFAM entries (*vide supra*) and Gene Ontology (GO, The Gene Ontology Consortium. "Gene ontology annotations and resources", Nucl. Acids Res. 2013, vol. 41, pp. D530-D535. The discarded GO terms affected the following categories: Solubility and relationship with the membrane, activity, bi-nding, polymer state and promiscuity in the protein binding.

The screening gave as result a list of 226 domains that was manually curated as it is described in the Result section to discard specific activities or properties that were not included in the databases commented above.

### Cultivation of mammal cells, vectors of expression and cellular analysis

Cells NIH3T3 and HEK293T were held in a DMEM medium (Dulbecco's modified Eagle's medium) containing glutamine supplemented with antibiotics and 10 % of FCS. The synthetic DNA coding the peptide tags were sub-cloned in a plasmid derived from pEGFP-N1 (Clontech) where some of the polylinker sequences had been replaced by T7 promoter, ribosome-binding sequences for gene expression in *E. coli* and in mammal cells, and a 6His peptide for affinity purification. Cells were transfected with Lipofectamine 2000 following the manufacturer's instructions (Invitrogen), and analyzed 24 hours after the transfection. The expression levels and aggregation index of the GFP fusion in the HEK293T cells were analyzed by epifluorescence microscopy with the help of the image analysis program ImageJ (Wayne Rasband, NIH).
Fluorescence loss in photobleaching (FLIP) was performed with a Zeiss LSM780 confocal microscope. A small circular region of the cytoplasm (3.6 µm²) was repeatedly photobleached at maximum laser power for 3 seconds per time and, between bleaching periods, the cell was examined by image with low intensity light to measure fluorescence loss. For the quantitative analysis, background intensity was subtracted, and the intensities outside the photobleaching area were measured over time and normalised to those of an unbleached transfected cell.

### Yeast strains, growth conditions and cell measurements

The parental yeast strain CML 128 and the methods used for gene replacement by recombination have been described in Gallego, C., et.al. "The Cln3 cyclin is downregulated by translational repression and degradation during the G1 arrest caused by nitrogen deprivation in budding yeast". EMBO J. 1997, vol. 16, pp. 7196-7206. Yeast cells were made to grow under exponential conditions for 7-8 generations in SC medium (Sambrook, J. & Russell, D. W. "Molecular cloning: A laboratory manual". 2001 3rd Edition, pp. 323-325, CSHL Press, Cold Spring Harbor, NY) with 2 % glucose at 30 °C, unless stated otherwise. The cell volume at budding time was determined from bright-field images with the help of a BudJ (Ferrezuelo, F. et al. "The critical size is set at a single-cell level by growth rate to attain homeostasis and adaptation". Nat. Comm. 2012, vol. 3, pp. (1012), an ImageJ plugin available at www.ibmb.csic.es \home\maldea.

### Purification of the peptide tags

The expression of monomeric tag ET5 (Phl P 2) labelled with the 6His hexapeptide, dimeric tag ET6 (Hev b 6.02), or of the corresponding fusions to GFP was induced in *E. coli* BL21 (DE3) with 0.5 mM IPTG and grown at 25 °C for 18h at 220 rpm. The expressed proteins were purified by affinity in Ni-NTA (Qiagen) according to the manufacturer's instructions.

### Monoclonal antibody production

Female BALB/c mice were intraperitoneally immunized with 75 µg of each one of the peptide tags of the invention, emulsified in adjuvant (Stimune Adjuvant; Prionics). 30 and 60 days later, the mice were injected with 75 µg of each one of the antigens in adjuvant. 4 days before the fusion procedure, each mouse received an intravenous injection of the same antigen in PBS 20 mM phosphate. The splenocytes were fused with myeloma cell line Sp2/0-Ag14. The hybridoma clones were selected by indirect conventional ELISA, and the cells from positive wells were cloned by limiting dilution. Monoclonal antibodies were purified from the supernatant by affinity chromatography in protein G (HiTrap Protein G Sepharose High Performance; GE Healthcare).

### Analysis by immunodetection on membrane

The analysis by immunodetection on membrane was performed with αGFP (clones of mouse 7.1 and 13.1, Roche), αCdc28 (polyclonal rabbit antibodies, ceded by C. Mann), and αYdj1 (clones of mouse 1G10.H8, Abnova). The solubility of the fusions of the tags of the invention with GFP was evaluated by centrifugating the cell extracts prepared by lysis buffer at 20,800 g for 15 minutes (50 mM Tris-HCl pH 8, 150 mM NaCl, 2 mM DTT and protease and phosphatase inhibitors).

### Immunofluorescence

HEK293T or NIH3T3 cells were quickly rinsed with PBS and fixed in 4% formaldehyde and 4% sucrose for 30 minutes at room temperature. Fixed cells were permeabilized with 0.1 % triton in PBS for 5 minutes, and blocked with 1% BSA in PBS. The hippocampal neuron block was performed with 5% goat serum in PBS. Primary antibodies against HA (rat clone 3F10 Roche), FLAG (mouse clone M2, Sigma), ET5 (mouse clone R19/8-11/18) and ET6 (mouse clone R19/4-11/15) were used with secondary antibodies labelled with Alexa568 (Molecular Probes) in a blocking solution. αGFP (mouse clones 7.1 and 13.1, Roche) was used as reference for the signal and background analysis.

### Results

### Initial bioinformatic screening for the selection of candidates

In order to identify polypeptides with a potential use as tag-proteins, with a minimal risk of physical or functional interference with the proteins of interest, a search using the Protein Data Bank (PDB) and Protein Families Database (Pfam) for protein domains with known three-dimensional structure and marked immunogenic capacity was performed. Due to the low reliability of the forecast on the antigenic properties, the starting point of the search was the experimentally confirmed immunogenic regions obtained from the Immune Epitope Database (IEDB) as well as of the Antigenic Variation Database (VarDB). Functional filters were applied to avoid the selection of insoluble, hydrolytic, polymeric or promiscuous domains, and the selection was focussed on those with a high globularity index, small size and large capacity as immunogens, based on a combination of physico-chemical factors. Consideration was also given to accessible surface area and flexibility in the N or C-terminal ends to facilitate the construction of the resulting fusion protein. These structures were filtered in a later round to discard all those domains that were scored as domains with enzymatic capacity or with capacity to bind cofactors or effectors. Domains with the capacity to form homo-polymers or with cellular regulatory, degradation or localization sequences were also rejected. Finally, domains from a vertebrate organism were discarded. The 12 resulting domains with potential to be developed as antigenic tags are listed in Table 1.

**Table 1 | Antigenic protein candidates.**

| Epitag | PDB id | Description | Organism | aa | Globularity |
|---|---|---|---|---|---|
| 1 | 1BK8 | Antimicrobial protein 1 | Horse chestnut (*Aesculus hippocastanum*) | 50 | 0.654 |
| 2 | 1BW3 | Barwin, basic seed protein | Barley (*Hordeum vulgare*) | 125 | 0.413 |
| 3 | 1ICX | Protein LLR18A | Yellow lupin (*Lupinus luteus*) | 155 | 0.450 |
| 4 | 1PJW | Envelope protein | Encephalitis virus | 111 | 0.389 |
| 5 | 1WHP | Allergen Phl p2 | Timothy grass (*Phleum pratense*) | 96 | 0.562 |
| 6 | 1WKX | Hevein isoform 2 | Rubber tree (*Hevea brasiliensis*) | 43 | 0.598 |
| 7 | 1X6R | Fimbrial protein | *Pseudomonas aeruginosa* | 123 | 0.507 |
| 8 | 2JMH | Mite allergen Blo t5 | Mite (*Blomia tropicalis*) | 119 | 0.371 |
| 9 | 2JTY | Type-1 fimbrial protein, A chain | *Escherichia coli* | 184 | 0.452 |
| 10 | 3BBG | Pollen allergen 5 | Great ragweed (*Ambrosia trifida*) | 40 | 0.587 |
| 11 | 3FT9 | Allergen Phl p3 | Timothy grass (*Phleum pratense*) | 100 | 0.568 |
| 12 | 3K78 | Major pollen allergen Bet v1-D/H | Birch (*Betula verrucosa*) | 160 | 0.424 |

### Biochemical viability tests of the labeling tags in vivo

Improperly folded proteins typically accumulate as aggregates that can be easily observed under the microscope as inclusion bodies. The biochemical alterations characterising aggregates normally include reduced solubility (in the cellular extracts), higher density and lower diffusion mobility (measured in a fluorescence microscope) and a lower accessibility to the antibodies (measured using immunofluorescence techniques). Thus, to evaluate whether the tags forming part of the invention contain clear structural determinants that allow them to fold quickly and efficiently, these were expressed at high concentrations as fusion proteins together with the GFP in embryonic kidney cells (HEK293T), and their propensity to form aggregates as well as their localization pattern during *in vivo* fluorescence microscopy experiments were analyzed (FIG. 1 a). Most of the fusions with the tags of the invention acquired expression levels similar to those of the GFP control experiment (FIG. 1b). Only one tag (ET8) was not detectable, and was not subsequently tested. With respect to their intracellular pattern (FIG. 1c), 3 of the tags analyzed in depth (ET1, ET2, and ET12) showed a marked accumulation in the cells, coupled with very low cytoplasm diffusion levels. 3 tags (ET7, ET9 and ET11) showed aggregates in the majority of the cells, although a significant fraction of the fusion protein produced a diffuse pattern in the cell. ET3 and ET4 showed a diffuse pattern in most cells, but they clearly showed aggregates in some of them. Finally, the 3 remaining domains (ET5, ET6 and ET10) produced a diffuse pattern in all cells that was indistinguishable from that produced in the experiment with GFP as control, suggesting that these 3 domains did not disturb the physico-chemical and biochemical properties of GFP *in vivo.* Therefore, these 3 domains were selected for subsequent testing.

Despite their small size, it was inferred that the structure of the three tags (ET5 corresponding to protein Phl P 2, ET6 corresponding to protein Hev b 6.02 and ET10 corresponding to protein Amb t 5) is based on a compact packaging based on beta sheets that, in the case of ET6 and ET10, is stabilised by the presence of disulphide bridges. The fusions of these three tags to GFP were expressed in the HEK293T cells and were detected basically as a single band with the expected apparent molecular weight for each case in western blot experiments (FIG.2d), and their stability in the presence of cycloheximide was confirmed as virtually identical to that of the control GFP (FIG. 2e). This data indicates that these three peptide tags do not significantly affect *per se* the integrity and degradation rates of the fusion protein.

The presence of any of these three tags did not affect the solubility of GFP in cellular extracts (FIG.2e), nor did it modify the *in vivo* intracellular diffusion rate as shown by the results of the fluorescence loss analysis (FIG.2f), thus confirming the absence of stable associations of the tags of the invention with cytoskeleton structures or other large size cell structures. Finally, it was concluded that these three tags do not affect either the maturation or the folding of GFP as a model protein of interest since neither the fluorescence nor protein levels were affected by the presence of any of the peptide tags of the invention.

### Functional safety of the labeling tags of the invention in model essential proteins

The fusion of labeling tags at the N- or C-terminal ends of the proteins of interest can produce unexpected effects in their functions, intracellular localization or association to macromolecular complexes, particularly if the tags do not have a clearly defined structure and are intrinsically disordered. The three tags forming part of the invention contain clear structural determinants and should therefore have less propensity to produce unexpected effects in the fusion proteins under test when compared with other labeling tags such as FLAG or HA, two of the more widely used tags. To test this last point, it was decided to perform a series of comparative analyses of the effects caused by tagging two essential proteins from *S*. *cerevisiae* yeast, a cyclin-dependent kinase and a J domain chaperone.

Cyclin-dependent kinases (Cdks) provide multiple interactions to carry out a series of processes triggering the cell cycle, and the modification of these interactions has a deep impact on their performance. Yeast depends on a single Cdk, Cdc28, to trigger all cell cycle phases in a coordinated manner with the cell growth machinery. The isolation of mutants with altered cell size has been crucial in the discovery of key molecular processes controlling the cell cycle and, at the same time, the cell size is a particularly valid indicator of molecular processes wherein Cdc28 plays a central role.

Thus, it was decided to tag the endogenous CDC28 locus with fusions at the C-terminal end with ET5 (Phl P 2 96 amino acid residues) and ET6 (Hev b 6.02, 43 amino acid residues) tags as well as FLAG tandem repeats (6x, 62 amino acid residues) and HA (3x, 32 amino acid residues) to obtain tags that were similar in size. None of these tags caused significant effects in Cdc28 levels or in its integrity (5A). However, as it is apparent from a visual examination of the yeast cells (FIG. 3B), Cdc28-FLAG fusion caused a significant change in cell size and shape, indicating important alterations in the signalling pathways mediated by Cdc28 in G1 and G2 phases of the cell cycle. Significantly, the same effects were observed when only 3 copies of the FLAG tag were used to label Cdc28 at the C-terminal end. This last result indicates that an intrinsic sequence determinant in the FLAG tag is responsible for the deleterious effects on the biological function of Cdc28. On the other hand, while ET5 has a size similar to the 6xFLAG tag, this did not produce any change in the morphology of the cells (FIG. 3B) and only a minimal effect in the mean critical size in the G1/S transition when compared with the 6xFLAG tag (FIG. 3c) was achieved. 3xHA tag did not give rise to large scale morphological defects, but it did induce a significant increase of the critical size in the G1/S transition (FIG. 3c), showing a clear alteration of the mechanisms that control the entry of the cellular cycle according to cell growth. On the contrary, ET6 tag (Hev b 6.02) (of size similar to 3xHA) did not cause significant changes in the average or critical size distribution values.

Molecular chaperones use the energy derived from the hydrolysis of ATP phosphate bonds to transmit conformational changes to their client proteins, thus facilitating their correct folding and promoting the assembly or disassembly of multi-protein complexes. One of the most ubiquitous types of chaperones is the Heat Shock 70 kDa (Hsp70s) family, which invariably requires the participation of J-domain co-chaperones, which stimulate their ATPase activity and cooperate in the binding of client proteins. Ydj1 is the most abundant J-domain chaperone in yeast and, apart from its activities in the homeostasis of the proteome, it plays a crucial role in the release of Cln3 cyclin of the endoplasmic reticulum to trigger the entry into the cellular cycle. In addition to its J-domain located at the N-terminal end, Ydj1 contains a dimerization domain at the C-terminal end that is essential for its functions. Consequently, it was decided to obtain fusions at the C-terminal end of the endogenous YDJ1 locus (FIG. 5b) and test possible deleterious effects of the peptide tags ET5 ET6, 6xFLAG and 3xHA on the function of Ydj1. None of the peptide tags caused detectable effects in the Ydj1 levels or in its integrity. In similar manner to Cdc28, the fusion of Ydj1 with the 6xFLAG tag produced the most severe effects, which could be observed even under normal conditions of growth at 30 degrees Celsius (FIG. 3e). Furthermore, both the 6xFLAG tag and the 3xHA tag produced a clear decrease in growth at high temperatures, where the function of the Ydj1 co-chaperone becomes essential. In clear contrast, the fusion of Ydj1 to ET5 or ET6 produced very moderate effects on growth at 37 degrees Celsius (FIG. 3e). Ydj1 is also very important for coordinating the growth and entry into the cell cycle associated to size. In line with their effects on the growth rate, both 6xFLAG tag and 3xHA tags caused a marked increase in the critical size in the G1/S transition (FIG. 3f). Quite on the contrary, neither ET5 nor ET6 produced an obvious effect on the critical size, which suggests that these labeling tags are functionally harmless in terms of the role played by Ydj1 in cell cycle entry processes.

### Labeling tags as safe tools for the analysis of proteins in-situ

The prospectuses that usually accompany commercially available monoclonal antibodies include information on their use for the detection of proteins of interest via immunofluorescence techniques *in-situ.* However, a credible proof is very rarely provided of their specificity in cells where the expression of the protein of interest has been decreased. This is one of the reasons why peptide tags such as FLAG or HAS are widely used for confirming the intracellular localization of the protein under very specific conditions. Taking this into account, the inventors have devised and implemented the efficient production of monoclonal antibodies for the *in-situ* detection of the labeling tags of the invention, using immunofluorescence techniques.

Both in monomer form (ET5, Phl P 2) or in dimeric form (ET6 corresponding to Hev b 6.02 and ET10 corresponding to WBA t 5), the tags and their corresponding GFP fusions were expressed and purified in *E. coli* cells (FIG. 6), to then immunize mice and perform an initial screening by using indirect ELISA methods. The monoclonal antibodies that came up positive were tested at low concentrations HIK293T cells by immunofluorescence to check their sensitivity and efficiency (FIG.7) and, above all, a low background signal at high concentrations in control cells (FIG.8). These antibodies are those that are generated by the hybridoma deposited at the institution "Leibniz-Institut DSMZ - Deutsche Sammlung von Mikroorganismen und Zellkulturen GmbH" with deposit numbers DSM ACC3236, DSM ACC3234 and DSM ACC3242. The selected monoclonal antibodies were subsequently used to analyze a series of fusions of ET5 and ET6 tags to paradigmatic proteins located in different cellular compartments of 3T3 fibroblasts and hippocampal mouse neurons (*Mus musculus*); FIG. 4 shows representative images. Regardless of the tag employed, all of the fusion proteins showed the expected intracellular localization and, what is more important, no protein aggregates or fractions were observed in abnormal compartments that could be interpreted as a consequence of the interaction of the peptide tags with the labelled proteins. In summary, these results demonstrate the functional safety of the tags of the invention with regard to the distribution of a diverse group of proteins that are located in different cell compartments such as the nucleus, the Golgi apparatus, cytoskeleton, endoplasmic reticulum, plasma membrane or the neuronal synapse.

### REFERENCES QUOTED IN THE APPLICATION

Terpe K. "Overview of tag protein fusions: from molecular and biochemical fundamentals to commercial systems" Appl. Microbiol Biotechnol. 2003, vol. 60., pp. 523-533
Stadler, C. et al. "Immunofluorescence and fluorescent-protein tagging show high correlation for protein localization in mammalian cells" Nat. Methods 2013, vol. 10, pp. 315-323
Altschul S. "Basic local alignment search tool", J. Mol. Biol. 1990, vol. 215, pp. 403-410
Hayes, C. N. et al. "varDB: a pathogen-specific sequence database of protein families involved in antigenic variation" Bioinformatics 2008, vol. 24, pp. 2564-2565
Vita, R. et al. "The immune epitope database 2.0" Nucl. Acids Res. 2010, vol. 38, D854-D862
Punta, M. et.al. "The Pfam protein families database". Nucl. Acids Res. 2012, vol. 40, D290-D301
Humphrey, et.al. "VMD - Visual molecular dynamics" J. Molec. Graphics 1996, vol. 14, pp. 33-38
GO, The Gene Ontology Consortium. "Gene ontology annotations and resources", Nucl. Acids Res. 2013, vol. 41, pp. D530-D535
Gallego, C., et.al. "The Cln3 cyclin is downregulated by translational repression and degradation during the G1 arrest caused by nitrogen deprivation in budding yeast". EMBO J. 1997, vol. 16, pp. 7196-7206
Sambrook, J. & Russell, D. W. "Molecular cloning: A laboratory manual". 2001 3rd edition, CSHL Press, Cold Spring Harbor, NY
Ferrezuelo, F. et al. "The critical size is set at a single-cell level by growth rate to attain homeostasis and adaptation". Nat. Comm. 2012, vol. 3, pp. 1012 Saha, S. & Raghava, G. P. S. "BcePred: prediction of continuous B-cell epitopes in antigenic sequences using physico-chemical properties" in Nicosia, G., Cutello, V., Bentley, P.J. & Timis, J. (eds.) pp. 197-204, Springer 2004, Heidelberg

## Claims

1. A recombinant fusion protein comprising a protein or peptide that is fused at its N-terminus or C-terminus end to a tag protein where the tag protein is defined according to the following properties:
1.1) it comprises a number of amino acid residues below 100;
1.2) it is immunogenic;
1.3) it has a globularity index above 0.5, defining the globularity index as the fraction of residues with solvent accessibility and calculated using the VMD program;
1.4) it is free from enzymatic activity;
1.5) it is free from binding sites for cofactors or effectors;
1.6) it is free of degradation sequences;
1.7) it is free of localization sequences;
1.8) it is of vegetable origin;
1.9) it is free of homo-polymerization or hetero-polymerization sequences;
1.10) when the N-terminal end is fused to the Green Fluorescent Protein and the fusion protein is expressed in HEK293T cells, the resulting recombinant fusion protein maintains the expression levels, wherein said levels are measured by immunodetection; it maintains the structural stability defined according to immunodetection levels for 12 hours of treatment with 100 µg/ml cycloheximide; it maintains intracellular diffusion *in vivo* as measured by fluorescence loss in photobleaching, fluorescence measured by confocal fluorescence microscopy; and it maintains the solubility of GFP in cell extracts by centrifugation of extracts measured at 15,000 g for 15 minutes, *in vitro* and *in vivo*;
1.11) when it is fused to the C-terminal end of protein Cdc28 or protein Ydj1 of *Saccharomyces cerevisiae* the recombinant fusion protein maintains the morphology defined by bright-field microscopy; and maintains cell viability measured by plating at 42°C;
1.12) when it is fused to the Green Fluorescent Protein and is expressed in *Escherichia coli,* it maintains the expression levels, wherein such levels are measured by immunodetection; it maintains the solubility of GFP in cell extracts measured by centrifuging extracts at 15,000 g for 15 minutes; and it maintains efficiency in purification by affinity chromatography measured by immunodetection;
1.13) it maintains the expression levels, wherein such levels are measured by immunofluorescence and maintains cell localization determined by confocal fluorescence microscopy of proteins that are found in different cell compartments, where such proteins are selected from the group consisting of: cytoskeleton beta-actin, protein STX6 of the Golgi apparatus, HO1 of the endoplasmic reticulum, HDAC2 nuclear and GRB2 of the plasma membrane in NIH3T3 mouse cells, as well as FMRP of the neuronal synapse in hippocampus neurons; and
wherein the tag-protein is selected from the group consisting of a fragment of Phl P 2 of *Phleum pratense* having an amino acid sequence with at least 70 % identity to SEQ ID NO: 1, a fragment of Hev b 6.02 of *Hevea brasiliensis* having an amino acid sequence with at least 70 % identity to SEQ ID NO: 2 and a fragment of Amb t 5 of *Ambrosia trifida* having an amino acid sequence with at least 70 % identity to SEQ ID NO: 3, or a combination thereof.

2. Recombinant fusion protein according to claim 1, wherein the tag-protein is a fragment of Phl P 2 of *Phleum pratense* having an amino acid sequence with at least 70 % identity to SEQ ID NO: 1.

3. The recombinant fusion protein according to Claim 1, wherein the tag-protein is a fragment of Hev b 6.02 of *Hevea brasiliensis* having an amino acid sequence with at least 70 % identity to SEQ ID NO: 2.

4. The recombinant fusion protein according to Claim 1, wherein the tag-protein is a fragment of Amb t 5 of *Ambrosia trifida* having an amino acid sequence with at least 70 % identity to SEQ ID NO: 3.

5. A nucleotide sequence that encodes for the recombinant fusion protein according to any one of Claims 1 to 4.

6. A recombination expression cassette which comprises the nucleotide sequence according to Claim 5 functionally linked to an expression control sequence.

7. The expression cassette according to Claim 6, wherein the control of the expression sequence comprises a target sequence for transcriptional regulators, a ribosome binding sequence and a transcription termination sequence.

8. An expression vector comprising the nucleotide sequence according to Claim 5 or an expression cassette according to Claim 6.

9. A host cell transfected or transduced with the nucleotide sequence according to Claim 5.

10. A method for obtaining a recombinant fusion protein according to any one of Claims 1-4, comprising:
1) Fusing at least one nucleotide sequence encoded for the protein or peptide with a nucleotide sequence encoded for the tag-protein;
2) Cloning the resulting sequence from step 1 in a host cell;
3) Expressing the sequence cloned in step 2 in a host cell.

11. A method for purifying or isolating a recombinant fusion protein according to any one of Claims 1-4, which comprises carrying out the steps according to Claim 10 and additionally a step wherein the fusion protein is purified or isolated.

12. The method according to Claim 11 wherein the additional purification or isolation step of the fusion protein comprises an antibody or fragment thereof that binds to the tag-protein.

13. A method for detecting a recombinant fusion protein according to any one of Claims 1-4, which comprises carrying out the steps according to Claim 10 and additionally a step wherein means are added for detecting the tag-protein.

14. The method according to Claim 13 wherein the means for detecting the tag-protein include an antibody or a fragment thereof.

15. The method according to Claim 14 wherein the means for detecting the tag-protein include a monoclonal antibody.

16. The method according to Claims 12-15 wherein the media are selected from antibodies that are obtained by the hybridoma deposited at the institution "Leibniz-Institut DSMZ - Deutsche Sammlung von Mikroorganismen und Zellkulturen GmbH" with deposit numbers DSM ACC3236, DSM ACC3234 and DSM ACC3242.

17. A monoclonal antibody that specifically binds to a fragment of Phl P 2 of *Phleum pratense* having an amino acid sequence with at least 70 % identity to SEQ ID NO:1, and which is produced by the hybridoma deposited at the institution "Leibniz-Institut DSMZ - Deutsche Sammlung von Mikroorganismen und Zellkulturen GmbH" with deposit number DSM ACC3234.

18. A monoclonal antibody that specifically binds to a fragment of Hev b 6.02 of *Hevea brasiliensis* having an amino acid sequence with at least 70 % identity to SEQ ID NO: 2, and which is produced by the hybridoma deposited at the institution "Leibniz-Institut DSMZ - Deutsche Sammlung von Mikroorganismen und Zellkulturen GmbH" with deposit number DSM ACC3242.

19. A monoclonal antibody that specifically binds to a fragment of Amb t 5 of *Ambrosia trifida* having an amino acid sequence with at least 70 % identity to SEQ ID NO: 3, and which is produced by the hybridoma deposited at the institution "Leibniz-Institut DSMZ - Deutsche Sammlung von Mikroorganismen und Zellkulturen GmbH" with deposit number DSM ACC3236.
